Europäisches Patentamt

(19) European Patent Office

Office européen des brevets

(11) Publication number: **0 000 292
B1**

# EUROPEAN PATENT SPECIFICATION

(45) Date of publication of patent specification:
**01.04.81**

(51) Int. Cl.³: **C 07 D 251/36, D 06 L 3/06**

(21) Application number: **78300115.9**

(22) Date of filing: **03.07.78**

(54) Production of chloro-s-triazine triones and their use in cleansing or bleaching compositions.

(30) Priority: **05.07.77 US 812556**

(43) Date of publication of application:
**10.01.79 Bulletin 79/1**

(45) Publication of the grant of the patent:
**01.04.81 Bulletin 81/13**

(84) Designated Contracting States:
**BE DE FR GB**

(56) References cited:
**US-A-3 453 274**

(73) Proprietor: **MONSANTO COMPANY, Patent Department 800 North Lindbergh Boulevard, St. Louis, Missouri 63166 (US)**

(72) Inventor: **Balaban, Michael Stephen, 15616 Century Lake Drive, Chesterfield Missouri (US)**
Inventor: **Cox, Raymond Cecil, Jr., 109 Stites Avenue, Belleville Illinois (US)**

(74) Representative: **Lunt, John Cooper et al, Monsanto House 10-18 Victoria Street, London, SW1H ONQ (GB)**

Production of chloro-s-triazine triones and their use in cleansing or bleaching compositions

The present invention relates to an improved process for manufacturing chloro-s-triazine triones which are sometimes referred to as chloro-cyanuric acids or chloroisocyanuric acids. More specifically, this invention pertains to a method for preparing chloro-s-triazine triones, particularly trichloro-s-triazine trione, having enhanced crystal properties.

The preparation of chloro-s-triazine triones such as trichloro-s-triazine trione or dichloro-s-triazine trione is well known in the prior art.

One method for producing chloro-s-triazine trione is described in U.S. Patent No. 2,969,360. In this process, cyanuric acid is fed along with aqueous alkali metal hydroxide (in molar ratio of about one mole of alkali metal hydroxide per atom of chlorine to be attached) and chlorine to an aqueous reaction zone which is maintained at a pH in the vicinity of 3.5. The feed ingredients are added in essentially stoichiometric proportions. The crude chloro-s-triazine trione precipitates from the solution forming a slurry. The slurry is continually or periodically filtered to separate the crystalline product from the mother liquor and the crystalline product is dried.

Prior art processes for producing chloro-s-triazine trione have been beset with numerous difficulties attributable to small particle size. For example, considerable variations in the rate of production have been experienced in the manufacture of trichloro-s-triazine trione due to difficulties in water removal which result in a slushy feed to the dryer. When very wet or slushy product material reaches the dryer it may become necessary to reduce the production rate or to shut down the unit to avoid packaging wet trichloro-s-triazine trione. The primary cause for this problem is believed to be the very fine particle size produced in the process.

The patent literature reports other problems attributable to small particle size such as those relating to product separation (filtration), washing and drying as well as those relating to handling of the final dusty product. Furthermore, it is thought that the crystal properties of the chloro-s-triazine triones have substantial influence on the retention of bleaching strength in formulations containing such triones. Thus, particle size and particle clarity are believed to be important for superior bleach stability.

It has been proposed heretofore in U.S. Patent No. 3,120,522 that chloro-s-triazine trione crystals having increased size can be produced by adding to the reaction mixture from which these crystals are formed, from 50 to 1,000 ppm of a chlorinated hydrocarbon containing 1 to 6 carbon atoms and having not more than one hydrogen atom in its molecule.

It has further been proposed in U.S. Patent No. 3,453,274 that the crystal size of chloro-s-triazine triones may be increased by adding, as a surface active agent, an alkali metal alkyl sulfate or an alkali metal alkylarylsulfonate to the reaction mixture while maintaining a pH between about 1.0 and 4.5.

U.S. Patent No. 3,941,784, teaches the crystal promotion of chloro-s-triazine trione by adding to the reaction mixture a small amount of polyoxyethylene, polyoxypropylene, or polyoxyethylene-polyoxypropylene copolymers.

In accordance with the present invention, chloro-s-triazine triones, in particular dichloro-s-triazine trione, trichloro-s-triazine trione and mixtures thereof, having enhanced crystal properties and exhibiting outstanding stability when formulated with other chemicals in bleaching products, are obtained by the use of certain crystal modifiers during the manufacture of chloro-s-triazine triones. These crystal modifiers are alkylated diphenyloxide disulfonic acids wherein the alkyl group contains from 8 to 14 carbon atoms, or their alkali metal salts. In these compounds, the sulfonic groups are attached to carbon atoms of the diphenyloxide nucleus.

It is preferred to add the crystal modifier to the reaction mixture in the form of the alkali metal disulfonate rather than in the acid form. The preferred alkali metal is sodium.

The mechanism by which the crystal modifiers used in this invention achieve superior bleach stability in the product is not fully understood. However, as indicated above, one of the factors involved is believed to be the crystalline form of the product and the mechanism is therefore regarded as one of crystal modification and the additive is referred to herein as a "crystal modifier".

Examples of crystal modifiers which can be used in the process of the invention are sodium octyl diphenyloxide disulfonate; sodium nonyl diphenyloxide disulfonate; sodium n-decyl diphenyloxide disulfonate; potassium n-decyl diphenyloxide disulfonate; sodium dodecyl diphenyloxide disulfonate; octyl diphenyloxide disulfonic acid; dodecyl diphenyloxide disulfonic acid; sodium tridecyl diphenyloxide disulfonate; and potassium tetradecyl diphenyloxide disulfonate. A preferred crystal modifier for use herein is sodium dodecyl diphenyloxide disulfonate. This disulfonate is commercially available. It can be obtained in liquid form as a 45–50% by weight concentrate, for example, from Dow Chemical Company under the trade name «Dowfax 2A1».The preferred concentration of the crystal modifier used in the process of the present invention is 20 to 500 parts per million (ppm) by weight based on the reactor contents. Improvements can be achieved with these crystal modifiers at higher or lower concentrations, however. Most preferred are concentrations of from 100 to 300 ppm by weight based on the reactor contents.

A preferred procedure for the production of trichloro-s-triazine trione by the process of the invention, comprises mixing a slurry of substantially pure cyanuric acid with alkali metal hydroxide (e.g., sodium or potassium hydroxide, preferably the former), to prepare an aqueous solution in which the alkali metal hydroxide to cyanuric acid molar ratio is about 3:1. The solution is then fed continuously to a reactor to which chlorine and the crystal modifier are also fed continuously, while maintaining the temperature of the reactor contents at about 25°C with a pH of about 3.5. Although the pH can vary between about 1.0 and 4.5, the range of 3.0 to 4.5 is preferable. The crystal modifier feed rate is adjusted to maintain the desired concentration of the crystal modifier in the reactor.

The product (trichloro-s-triazine trione) is withdrawn from the reaction as a slurry, then filtered, dried and packaged. When produced in this manner, the particles of trichloro-s-triazine trione are single, clear crystals of suitable size and structural integrity to exhibit outstanding bleach stability when formulated with other chemicals in bleaching and scouring compositions.

In certain circumstances it may be advantageous, in the above-described procedure, for the reactor to contain initially a small volume of water having at least a portion of the crystal modifier charge in solution.

It may also be desirable to introduce certain antifoaming agents during a continuous manufacturing process in order to offset any tendencies of the crystal modifiers to generate foam.

For certain applications, dichloro-s-triazine trione is the desired end product instead of trichloro-s-triazine trione. The former can be prepared in a manner similar to that described above except that the feed solution can be prepared by mixing a cyanuric acid slurry with an alkali metal hydroxide to produce a solution having a hydroxide to cyanuric acid mole ratio of about 2.1:1. Chlorine is typically introduced at a rate sufficient to maintain a pH in the range 2.1 to 2.3.

In drying the product of the process of the present invention, control of the drying conditions as for the drying of conventionally-produced chloro-s-triazine triones should be exercised. Desirably, trichloro-s-triazine trione should not be dried at temperatures which will cause the particle temperature to exceed about 130°C.

An abrupt absorption of heat into the trichloro-s-triazine trione particle is usually observed when the particle temperature during the drying step is allowed to exceed about 130°C. The phenomenon associated with this temperature is sometimes referred to as "phase change". Exceeding 130°C particle temperature during drying of trichloro-s-triazine trione is generally accompanied by a reduced density of the dried particle after cooling. Furthermore, the reduced density of the particle after cooling is characterized by extension of the lattice in the crystalline structure of the particle. It is therefore desirable to conduct the drying step associated with the process of this invention in a manner which maintains particle temperature during drying between about 80°C and about 120°C, preferably about 95°C to about 105°C.

The invention is illustrated by Example 3 of the following Examples. Examples 1 and 2 are comparative Examples. Parts and percentages are by weight unless otherwise specified.

Example 1
This Example illustrates a conventional preparation of trichloro-s-triazine trione wherein no crystal modifier or promoter is employed. A feed solution was prepared by mixing a cyanuric acid slurry with sodium hydroxide to produce a solution containing 7.6% cyanuric acid with a mole ratio of sodium hydroxide to cyanuric acid of 3.2:1. The chlorination reaction was provided for by a jacketed 1.4 litre glass reactor equipped with a stirrer, side arm for product overflow, subsurface feed tube and a fritted glass sparger. Starting with water in the reactor, feed solution was introduced through the feed tube at about 40 ml per minute and chlorine was introduced through the sparger at about 5.5 grams per minute. The pH was controlled in the range 3.5 to 3.8 by adjusting the chlorine feed rate, and the reaction temperature was controlled between 22° and 27°C by circulating ice water through the reactor jacket. The product slurry, which overflowed the side arm, was filtered to separate the crystalline product from the mother liquor, giving a filter cake containing 10 to 12% by weight free moisture, and was then dried in an oven at about 100°C. (The product was observed to settle slowly from the slurry.)

Example 2
This Example was conducted in a manner identical to that of Example 1 except for the presence of a crystal promoter within the scope of aforementioned US. Patent No. 3,941,784. The promoter employed was a polyoxyethylene-polyoxypropylene copolymer identified as «Pluronic L-62» and available from BASF-Wyandotte Corporation. («Pluronic» is a Registered Trade Mark at least in the United Kingdom.) A feed solution identical to that of Example 1 was prepared. A chlorination was conducted as described in Example 1 except that 200 ppm (based upon the reactor contents) of polyoxyethylene-polyoxypropylene copolymer was introduced to the reaction mixture. Part of this 200 ppm promoter addition was admitted to the initial reactor water charge and part was admitted to the feed solution. The resulting product in this case was observed to settle rapidly and was filtered to 4–5% free moisture.

Example 3
This Example illustrates the preparation of trichloro-s-triazine trione with the aid of a crystal modifier in accordance with the present invention. Example 3 was conducted in a manner identical to that of Example 1 except for the addition of 200 ppm, based upon the reactor contents, of sodium dodecyl-diphenyloxide disulfonate (as a 45% concentrate in a liquid vehicle) to the reaction vessel. The resulting trichloro-s-triazine trione product exhibited outstanding clarity in the single clear crystals which were produced.

Example 4

Dichloro-s-triazine trione can be prepared by a procedure similar to that of Example 3 using a feed solution prepared by mixing a cyanuric acid slurry with sodium hydroxide to produce a solution containing about 9.8% of cyanuric acid and having a sodium hydroxide to cyanuric acid mole ratio of about 2.1:1, and introducing chlorine at about 7.1 grams per minute to maintain a pH in the range 2.1 to about 2.3.

Example 5

In this comparative Example, trichloro-s-triazine trione was prepared by a procedure similar to that of Example 3, but using 200 ppm of a sodium $(C_{14}$ alkyl)benzene sulfonate, an example of the alkali metal alkylarylsulfonates disclosed in U.S. Patent 3,453,274, instead of sodium dodecyl diphenyloxide disulfonate.

Particle size comparisons were made on the respective dried products from Examples 1, 2, 3 and 5 above. In a first test on the products of Examples 1, 2 and 3, apparent particle size, on a relative basis, was ascertained by observing the cumulative weight percent of product retained on a screen having a predetermined number of meshes per unit length.

The unmodified, unpromoted product of Example 1 exhibited the smallest apparent particle size. The product of Example 2, although exhibiting an apparent size greater than that of Example 3 in screen measurements, is suspected of undergoing size attrition during certain conditions of handling in compounding operations sometimes employed to formulate cleansing and bleaching compositions referred to hereafter. Moreover, it will be seen from the chlorine stability testing described below that apparent particle size is not the sole factor determining stability.

In a second test, the relative particle sizes of the dried products of Examples 3 and 5 were determined using a screen having a mesh aperture of 0.074 mm. 95 percent of the product of Example 3 was retained on the screen, whereas only 62 percent of the product of Example 5 was retained, showing that the product made according to the process of the invention had a significantly lower percentage of particles smaller than 0.074 mm.

Bleach stability of cleansing compositions containing chloro-s-triazine triones is customarily determined by measuring the percentage of available chlorine remaining in the cleansing composition following a predetermined number of days exposure of the composition to ambient conditions. One such aging test calls for the placement of the cleansing composition in half-filled canisters and exposing the canisters to air at 80°F (26.7°C) and 80% relative humidity with both open and closed tops on the canisters.

Chlorine stability results with the product of Example 1 were substandard and unacceptable for commercial cleansing compositions. The following Table compares the chlorine stability of the products of Example 2 and Example 3. It will be seen that the single clear crystal of the product of Example 3, although of smaller apparent particle size than that of Example 2 according to screen measurements, is nonetheless a superior product from the standpoint of chlorine stability. The data in the Table were obtained following the typical aging test described above, using open top canisters, using typical cleansing formulations containing 80–85 percent of an abrasive and 0.5–1 percent of the trichloro-s-triazine trione, the balance being an alkaline builder.

| Formulation Exposure (days) | Available Chlorine Remaining (%) | |
| --- | --- | --- |
| | Example 2 | Example 3 |
| 8 | 78 | 82 |
| 10 | 69 | 79 |
| 12 | 61 | 77 |
| 14 | 54 | 74 |
| 16 | 47 | 72 |
| 18 | 40 | 70 |

From these results, it can be seen that the use of sodium dodecyl diphenyloxide disulfonate according to the process of the invention contributes significantly to available chlorine stability of the product.

**Claims**

1. A process for the production of dichloro-s-triazine trione, trichloro-s-triazine trione or a mixture thereof by the reaction of cyanuric acid with an alkali metal hydroxide and chlorine in an aqueous reaction mixture at a pH between about 1.0 and 4.5 containing a substance ("crystal modifier") which improves the form of the chloro-s-triazine trione product which precipitates from the reaction mixture, and recovering the chloro-s-triazine trione product from the reaction mixture, characterized in that the crystal modifier is an alkali metal disulfonate of an alkylated diphenyloxide wherein the alkyl group contains from 8 to 14 carbon atoms, or an alkylated diphenyloxide disulfonic acid wherein the alkyl group contains from 8 to 14 carbon atoms.

2. A process according to Claim 1 characterized in that the crystal modifier concentration in the aqueous reaction mixture is from 20 to 500 parts per million by weight.

3. A process according to Claim 2 characterized in that the crystal modifier concentration in the aqueous reaction mixture is from 100 to 300 parts per million by weight.

4. A process according to any of Claims 1 to 3 characterized in that the crystal modifier is a sodium disulfonate of a said alkylated diphenyloxide.

7 0 000 292 8

5. A process according to any of Claims 1 to 3 characterized in that the crystal modifier is sodium dodecyl diphenyloxide disulfonate, sodium n-decyl diphenyloxide disulfonate, or dodecyl diphenyloxide disulfonic acid.

6. A cleansing or bleaching composition containing a chloro-s-triazine trione characterized in that the chloro-s-triazine trione is dichloro-s-triazine trione, trichloro-s-triazine trione or a mixture thereof that has been produced by a process according to any of Claims 1 to 5.

**Patentansprüche**

1. Verfahren zur Herstellung von Dichlor-s-triazin-trion, Trichlor-s-triazin-trion, oder einer Mischung dieser Verbindungen, durch Umsetzen von Cyanursäure mit einem Alkalimetallhydroxid und Chlor in einer wässerigen Reaktionsmischung bei einem pH-Wert zwischen etwa 1,0 und 4,5, die eine Substanz («Kristallmodifiziermittel») enthält, welche die Form des Chlor-s-triazin-trion-Produkts, das aus der Reaktionsmischung ausfällt, verbessert, und Gewinnen des Chlor-s-triazin-trion-Produkts aus der Reaktionsmischung, dadurch gekennzeichnet, dass das Kristallmodifiziermittel ein Alkalimetalldisulfonat eines alkylierten Diphenyloxids, in welchem die Alkylgruppen 8 bis 14 Kohlenstoffatome enthalten.

2. Verfahren nach Anspruch 1, dadurch gekennzeichnet, dass die Konzentration des Kristallmodifiziermittels in der wässerigen Reaktionsmischung 20 bis 500 Teile pro Million Teile, bezogen auf das Gewicht, beträgt.

3. Verfahren nach Anspruch 2, dadurch gekennzeichnet, dass die Konzentration des Kristallmodifiziermittels in der wässerigen Reaktionsmischung 100 bis 300 Teile pro Million Teile, bezogen auf das Gewicht, beträgt.

4. Verfahren nach einem der Ansprüche 1 bis 3, dadurch gekennzeichnet, dass das Kristallmodifiziermittel ein Natriumdisulfonat eines vorerwähnten alkylierten Diphenyloxids ist.

5. Verfahren nach einem der Ansprüche 1 bis 3, dadurch gekennzeichnet, dass das Kristallmodifiziermittel Natriumdodecyl-diphenyloxiddisulfonat, Natrium-n-decyl-diphenyloxiddisulfonat, oder Dodecyl-diphenyloxiddisulfonsäure ist.

6. Reinigungs- oder Bleichzubereitung mit einem Gehalt an Chlor-s-triazin-trion, dadurch gekennzeichnet, dass das Chlor-s-triazin-trion Dichlor-s-triazin-trion, Trichlor-s-triazin-trion, oder eine Mischung daraus ist, die nach einem Verfahren gemäss einem der Ansprüche 1 bis 5 hergestellt worden sind.

**Revendications**

1. Procédé pour la production de dichloro-s-triazine trione, de trichloro-s-triazine trione ou d'un mélange de ces produits par la réaction d'acide cyanurique avec un hydroxyde de métal alcalin et du chlore dans un mélange réactionnel aqueux à un pH compris entre environ 1,0 et 4,5, contenant une substance («Produit de modification cristalline») qui améliore la forme de la chloro-s-triazine trione produite qui précipite à partir du mélange réactionnel, et la récupération de la chloro-s-triazine trione produite à partir du mélange réactionnel, caractérisé en ce que le produit de modification cristalline est un disulfonate de métal alcalin d'un oxyde de diphényle alkylé où le groupe alkyle contient 8 à 14 atomes de carbone, ou un acide oxyde de diphényle alkylé disulfonique où le groupe alkyle contient 8 à 14 atomes de carbone.

2. Procédé selon la revendication 1, caractérisé en ce que la concentration du produit de modification cristalline dans le mélange réactionnel aqueux est 20 à 500 parties par million en poids.

3. Procédé selon la revendication 2, caractérisé en ce que la concentration du produit de modification cristalline dans le mélange réactionnel aqueux est 100 à 300 parties par million en poids.

4. Procédé selon l'une quelconque des revendications 1 à 3, caractérisé en ce que le produit de modification cristalline est un disulfonate de sodium d'un oxyde de diphényle alkylé.

5. Procédé selon l'une quelconque des revendications 1 à 3, caractérisé en ce que le produit de modification cristalline est le dodécyloxyde de diphényle disulfonate de sodium, le n-décyloxyde de diphényle disulfonate de sodium ou l'acide dodécyloxyde de diphényle disulfonique.

6. Composition de nettoyage ou de blanchiment contenant une chloro-s-triazine trione, caractérisée en ce que la chloro-s-triazine trione est la dichloro-s-triazine trione, la trichloro-s-triazine trione ou un mélange de ces produits qui a été fourni par un procédé selon l'une quelconque des revendications 1 à 5.

5